# EUROPEAN PATENT APPLICATION

(11) **EP 4 775 253 A1**
(43) Date of publication of application: **15.07.2026**
(21) Application number: 23951205.6
(22) Date of filing: 08.09.2023
(51) Int. Cl.: A61M 25/10

(54) **BALLOON CATHETER SYSTEM HAVING REPAIR FUNCTION**

(71) Applicant: Hangzhou Matrix Medical Technology Co., Ltd., Hangzhou, Zhejiang 311100 (CN)
(72) Inventor: ZHANG, Chenguang, Hangzhou, Zhejiang 311100 (CN); JI, Peihong, Hangzhou, Zhejiang 311100 (CN); WANG, Yudong, Hangzhou, Zhejiang 311100 (CN)
(74) Representative: Metida
(86) International application number: PCT/CN2023/117757
(87) International publication number: WO 2025/050385

(57) **Abstract**

The present disclosure discloses a balloon catheter system with repair function, including: a tube body, having a distal end and a proximal end opposite to the distal end, and the tube body at least forming a fluid channel and an optical fiber channel; a balloon body, located at an outer periphery of the distal end of the tube body and communicating with the fluid channel; an optical fiber, arranged in the optical fiber channel and having a light emitting section extending to a position of the balloon body; and a laser generator, connected to a proximal end of the optical fiber and emitting a laser having a repair function with a wavelength ranging from 400 to 750 nm. The balloon catheter system provided by the present disclosure can effectively repair the tearing site of the vascular wall.

## Description

### TECHNICAL FIELD

The present disclosure relates to the field of medical device technology, and more particularly, relates to a balloon catheter system with repair function.

### DESCRIPTION OF THE PRIOR ART

Percutaneous intervention has become one of the commonly used treatment techniques worldwide. Balloon dilation and stent implantation are usually used for the treatment of intravascular stenosis lesions, however, both methods have their own shortcomings.

### SUMMARY OF THE DISCLOSURE

### TECHNICAL PROBLEMS

A possibility of restenosis after the treatment is high, when bare balloon dilatation is used for the treatment of intravascular stenosis. If bare metal stent catheter intervention is used for the treatment of intravascular stenosis, although the restenosis does not occur easily after the treatment, it may easily cause neointimal proliferation (i.e., the formation of scar tissue at a stent section). Once the restenosis or the neointimal proliferation occurs, another intervention treatment is required.

### TECHNICAL SOLUTIONS

The objective of the present disclosure is to provide a balloon catheter system with repair function, which can solve the problems of restenosis and neointimal hyperplasia after the interventional treatment.

The present disclosure provides technical solutions as follows:
a balloon catheter system with repair function, including:
a tube body, having a distal end and a proximal end opposite to the distal end, and the tube body at least forming a fluid channel and an optical fiber channel;
a balloon body, located at an outer periphery of the distal end of the tube body and communicating with the fluid channel;
an optical fiber, arranged in the optical fiber channel and having a light emitting section extending to a position of the balloon body; and
a laser generator, connected to a proximal end of the optical fiber and emitting a laser having a repair function with a wavelength ranging from 400 to 750 nm.

The present disclosure further provides a plurality of optional manners hereinafter, which are not intended to be additional limitations on the above-mentioned general solution, but are merely further additions or optimizations. On a premise that there is no technical or logical contradiction, each optional manner may be combined individually for the above-mentioned overall solution, or may be combined among multiple optional manners.

Optionally, a power of the laser emitted by the laser generator ranges from 10 to 30 mW.

In some embodiments, the laser having a predetermined wavelength is emitted by the laser generator in the following sequence in each working cycle:
in an initial stage, the laser has a power ranging from 5 to 8 mW/cm² and lasts for 10 to 20 s;
in an intermediate stage, the laser has a power ranging from 10 to 30 mW/cm² and lasts for 120 to 180 s; and
in a final stage, the laser has a power ranging from 5 to 8 mW/cm² and lasts for 10 to 20 s.

Optionally, the balloon body includes an isodiametric section and two tapered sections respectively located at both ends of the isodiametric section, and a surface of each tapered section at a distal end of the balloon body is covered with a black coating.

Optionally, a refractive index of the high refractive index ultraviolet adhesive ranges from 1.50 to 1.70.

Optionally, high refractive index ultraviolet adhesive is attached to an outer periphery of the balloon body distributed at intervals, and the high refractive index ultraviolet adhesive protrudes from a surface of the balloon body at a height ranging from 0.20 to 0.25 mm.

Optionally, the high refractive index ultraviolet adhesive is regularly distributed on the outer periphery of the balloon body.

Optionally, the high refractive index ultraviolet adhesive is arranged in the form of dot-shaped protrusions in a matrix, and a distance between two adjacent spots ranges from 3 to 10 mm.

Optionally, the high refractive index ultraviolet adhesive is arranged in the form of linear protrusions extending along an axial direction of the balloon body and equidistantly distributed around a circumference of the balloon body, and a number of the linear protrusions ranges from 3 to 10.

Optionally, a length of the linear protrusions matches a length of the isodiametric section.

Optionally, the tube body includes an inner tube and an outer tube mounted around the inner tube, the outer tube of the tube body is black, and the inner tube of the tube body is colorless and transparent; the inner tube of the tube body has a hardness ranging from 35 to 75D, and the hardness of the inner tube increases continuously or at intervals from the distal end to the proximal end of the tube body by a predetermined value.

Optionally, the inner tube of the tube body includes at least two first unit sections connected in sequence along an axial direction of the tube body, and for two adjacent first unit sections, a hardness of the first unit section closer to the distal end of the tube body is less than a hardness of the first unit section closer to the proximal end of the tube body.

Optionally, a number of the first unit sections is 2 to 4.

Optionally, a diameter of each first unit section is the same as each other; or, for two adjacent first unit sections, a diameter of the first unit section closer to the distal end of the tube body is less than a diameter of the first unit section closer to the proximal end of the tube body.

Optionally, an axial length of the first unit section closest to the proximal end of the tube body ranges from 20 to 30 cm.

Optionally, each first unit section adopts a single-layer tube or a multi-layer tube, the single-layer tube is made of one selected from PEBAX, nylon, or TPU; the layers of the multi-layer tube can slide relatively to each other, and each layer is independently made of PEBAX, nylon, or PE.

Optionally, the inner tube of the tube body includes a proximal inner tube and a distal inner tube connected to the proximal inner tube along the axial direction of the tube body; a hardness of the proximal inner tube ranges from 55 to 75D, and a hardness of the distal inner tube ranges from 35 to 55 D.

Optionally, the outer tube of the tube body includes at least two second unit sections connected in sequence along the axial direction of the tube body; for the two adjacent second unit sections, a hardness of the second unit section closer to the distal end of the tube body is less than that of the second unit section closer to the proximal end of the tube body.

Optionally, a number of the second unit sections is 2 to 4.

Optionally, a diameter of each second unit section is the same as each other; or, for the two adjacent second unit sections, a diameter of the second unit section closer to the distal end of the tube body is less than that of the second unit section closer to the proximal end of the tube body.

Optionally, each second unit section adopts a single-layer tube or a multi-layer tube, the single-layer tube is made of one selected from PEBAX or nylon; the layers of the multi-layer tube can slide relatively to each other, and each layer is independently made of PEBAX or nylon.

Optionally, an end portion of a distal end of the inner tube is welded with an elastic member, and the elastic member includes a bare section and a welding section arranged along the axial direction of the tube body; a ratio of an axial length of the bare section to that of the welding section ranges from 1:0.8 to 1:1.2, and a diameter of the elastic member gradually increases from a distal end of the bare section to a proximal end of the welding section.

Optionally, the elastic member is a spiral spring formed by spirally winding a metal wire with a diameter ranging from 0.04 to 0.1 mm.

Optionally, the metal wire is made of one selected from platinum-tungsten, platinum-iridium, stainless steel, gold, and nickel-titanium.

Optionally, a thread pitch of the bare section is equal to a diameter of the metal wire, and a thread pitch of the welding section is 1 to 3 times the diameter of the metal wire.

Optionally, an axial length of the elastic member ranges from 2 to 5 mm.

Optionally, an axial length of the bare section of the elastic member ranges from 0.5 to 2mm, an axial length of the welding section ranges from 1 to 3 mm.

Optionally, a diameter of the bare section ranges from 0.50 to 0.70 mm, and a diameter of the welding section ranges from 0.60 to 1.0 mm.

Optionally, a ratio of a distal end of the bare section to that of a proximal end of the welding sections ranges from 1:1 to 1:1.5.

Optionally, the proximal end of the welding section forms a welding ring welded to the wall of the inner tube by bending the metal wire forming the spiral spring.

Optionally, the tube body includes an inner tube and an outer tube mounted around the inner tube, a distal end of the balloon body is in sealed connection with an outer wall of the inner tube, and a proximal end of the balloon body in sealed connection with the outer tube, a radial gap between the inner tube and the outer tube forms the optical fiber channel, and each optical fiber is arranged in parallel with the inner tube or by wrapping around the inner tube.

Optionally, the balloon catheter system includes a plurality of the optical fibers, and the optical fibers are uniformly distributed around an axis of the tube body,.

Optionally, a diameter of each optical fiber ranges from 0.125 to 0.25 mm.

Optionally, the tube body includes an inner tube and an outer tube mounted around the inner tube, a distal end of the balloon body is in sealed connection with the outer wall of the inner tube, and a proximal end of the balloon body is in sealed connection with the outer tube; a radial gap between the inner tube and the outer tube forms a fluid channel, and the inner tube lumen forms the optical fiber channel.

Optionally, at least one section of the tube body along an axial direction thereof is a double-lumen tube, and the double-lumen tube includes the fluid channel and a guide wire channel isolated from the fluid channel, and the fluid channel communicates with the radial gap between the inner tube and the outer tube.

Optionally, a first guide wire port is formed in a tube wall of a portion of the inner tube extending out of a distal end of the balloon body; two ends of the guide wire channel respectively form a second guide wire port and a third guide wire port; the guide wire passes through the first guide wire port, the second guide wire port, and the third guide wire port, and a portion of the guide wire between the first guide wire port and the second guide wire port is located outside the balloon body.

Optionally, an outer surface of the balloon body is coated with at least one selected from an anti-proliferative drug, a drug that induce collagen or elastin cross-linking, and an anti-vasospasm drug.

Optionally, the drug is paclitaxel or rapamycin.

Optionally, the balloon body is provided with an implantable vascular stent on the outer surface thereof

### BENEFICIAL EFFECT

The balloon catheter system provided by the present disclosure concentrates the tearing of the vascular wall in a predetermined area and provides the laser having a specific wavelength to effectively repair the tearing in the vascular wall in the predetermined area, which can effectively avoid the occurrence of restenosis. When a vascular stent is implanted, the balloon catheter system of the present disclosure can also avoid neointimal proliferation.

### BRIEF DESCRIPTION OF DRAWINGS

FIG. 1 is a schematic view of a balloon catheter system with repair function in accordance with an embodiment of the present disclosure;
FIG. 2 is a schematic view of a high refractive index ultraviolet adhesive on a surface of a balloon body in a first distribution;
FIG. 3 is a schematic view of the high refractive index ultraviolet adhesive on the surface of the balloon body in a second distribution;
FIG. 4 is a cross sectional view showing a position relationship between an optical fiber and an inner tube;
FIG. 5 is a schematic view illustrating a first position relationship between the optical fiber and the inner tube;
FIG. 6 is a schematic view illustrating a second position relationship between the optical fiber and the inner tube;
FIG. 7 is a schematic view of an outer tube of the tube body in accordance with an embodiment of the present disclosure;
FIG. 8 is a schematic diagram of a balloon catheter system with repair function, in accordance with an embodiment of the present disclosure, wherein a guide wire is located outside the balloon body;
FIG. 9 is an enlarged view of a part A in FIG. 8;
FIG. 10 is a schematic view illustrating positions of the tube body, a double-lumen tube and the balloon body in FIG. 8; and
FIG. 11 is a cross-sectional view of the double-lumen tube in accordance with an embodiment of the present disclosure.

### DESCRIPTION OF REFERENCE NUMERALS

100, balloon catheter system; 110, balloon body; 111, dot-shaped protrusion; 112, linear protrusion; 113, isodiametric section; 114, tapered section; 120, optical fiber; 130, tube body; 131, outer tube; 132, inner tube; 133, double-lumen tube; 134, first guide wire port; 135, second guide wire port; 136, third guide wire port; 137, guide wire channel; 138, fluid channel; 140, catheter adapter; 150, stress release tube; 160, optical fiber joint; 170, elastic member; 171, bare section; 172, welding section; 173, welding ring; and 180, guide wire.

### DESCRIPTION OF EMBODIMENTS

The following clearly and completely describes the technical solutions in the embodiments of this application with reference to the accompanying drawings in the embodiments of the present disclosure. The described embodiments are only a part rather than all of the embodiments of the present disclosure. All other embodiments obtained by a person of ordinary skill in the art based on the embodiments of the present application without creative efforts shall fall within the protection scope of the present disclosure.

In order to better describe and illustrate the embodiments of the present application, one or more drawings can be referred to. However, the additional details or examples used to describe the drawings should not be considered as a limitation on the scope of any of the inventions, the embodiments or preferred modes of the present disclosure.

It should be noted that when a component is said to be "connected" to another component, it can either be directly connected to another component or there may be a intervening component. When a component is considered to be "placed on" another component, it can either be placed directly on another component or there may be an intervening component.

Unless otherwise defined, all technical and scientific terms used in this document have the same meanings as those generally understood by those skilled in the art to which the present disclosure pertains. The terms used in the specification of the present disclosure are solely for the purpose of describing specific embodiments and are not intended to limit the present disclosure.

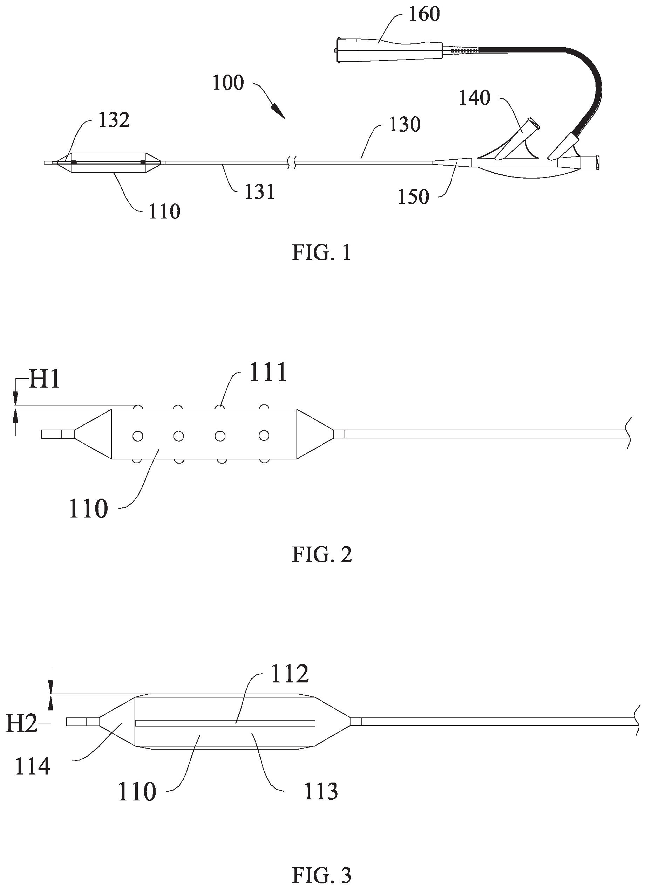

As shown in FIG. 1, a balloon catheter system 100 with repair function is provided in accordance with an embodiment of the present disclosure, including:
a tube body 130 having a distal end and a proximal end opposite to the distal end, and at least forming a fluid channel and an optical fiber channel;
a balloon body 110 located at an outer periphery of the distal end of the tube body 130 and communicating with the fluid channel;
at least one optical fiber 120 arranged in the optical fiber channel and having a light emitting section extending to a position of the balloon body 110; and
a laser generator connected to a proximal end of the optical fiber 120 and emitting laser having a repair function, wherein a wavelength of the laser ranges from 400 to 750 nm.

When the balloon catheter system 100 with repair function is used, firstly, fluid is injected into the balloon body 110 through the fluid channel of the tube body 130 to fill the balloon body 110. Then, a laser with a wavelength ranging from 400 to 750 nm is emitted by the laser generator. The laser is emitted from the light emitting section of the optical fiber 120 and is applied to a site to be repaired after passing through the balloon body 110 and the fluid inside the balloon body 110 and being scattered by the balloon body 110. Generally, continuous application of the laser for a predetermined period of time can achieve an ideal treatment effect.

High refractive index ultraviolet adhesive is attached to an outer periphery of the balloon body 110 and distributed at intervals. The high refractive index ultraviolet adhesive is regularly distributed on an outer surface of the balloon body 110. When the balloon body 110 is filled with fluid, the balloon body 110 expands to dilate the vascular wall of the human body. During the dilation of the vascular wall, a local tearing of the structure of the vascular wall may occur to achieve the dilation of the vascular wall. Since the refractive index ultraviolet adhesive protrudes from the outer surface of the balloon body 110, the tearing of the vascular wall is concentrated in areas where the high refractive index ultraviolet adhesive is distributed; when the high refractive index ultraviolet adhesive acts on the vascular wall, a stress concentration area in the vascular wall is more easily formed. By means of the arrangement of the high refractive index ultraviolet adhesive, the tearing of the vascular wall which is originally in disordered distribution is concentrated in the areas where the high refractive index ultraviolet adhesive is distributed. Meanwhile, in these areas, due to the enrichment effect of the high refractive index ultraviolet adhesive on the laser, the laser acts on the tearing area of the vascular wall more intensively, thereby completing the repairing of the tearing area.

With the high refractive index ultraviolet adhesive protruding from the outer surface of the balloon body 110, the technical solution of the present disclosure can concentrate the inevitably-disordered tearing of the vascular wall in specific areas where the high refractive index ultraviolet adhesive is distributed. At the same time, based on the enrichment effect of the high refractive index ultraviolet adhesive on the laser in the specific areas, the tearing of the vascular wall can be repaired more effectively.

The laser acts on the site to be repaired, which can promote the repairing of an injured blood vessel and effectively prevent the occurrence of restenosis.

The laser generator emits low-power laser in pulses or continuously emits the low-power laser, which is conducted into the balloon body 110 through the optical fiber 120. The light emitting section of the optical fiber 120 located inside the balloon body 110 applies the low-intensity laser to the site to be repaired. The low-intensity laser has a photo-modulating effect, which is generated by a photochemical effect rather than a thermal effect, thus, the low-intensity laser can promote the repairing of cells and tissues, accelerate the clearance of inflammatory mediators, and promote the absorption of tissue edema.

After the balloon catheter dilation, the inner wall of the blood vessel is easily injured. The red light of a predetermined wavelength emitted by the light emitting section of the optical fiber 120, which is applied to the injury site of the inner wall of the blood vessel after being scattered by the balloon body 110, can promote vascular endothelial cell proliferation and anti-inflammatory apoptosis, accelerate the blood vessel to return to a stable state, and prevent vascular restenosis after the balloon catheter dilation.

The laser emitted by the laser generator has a wavelength ranging from 400 to 750 nm. The laser appears red within the wavelength range, which can promote the repairing of cell nuclear tissues, and accelerate the clearance of inflammatory mediators and the absorption of tissue edema. In some embodiments, the laser emitted by the laser generator has a wavelength ranging from 630 to 700 nm.

The laser emitted by the laser generator has a power ranging from 10 to 30 mW. In some embodiments, the laser emitted by the laser generator has a power ranging from 10 to 15mW.

The laser having a predetermined wavelength is emitted from the laser generator in the following sequence in each working cycle:
in an initial stage, the laser has a power ranging from 5 to 8 mW/cm² and lasts for 10 to 20 s;
in an intermediate stage, the laser has a power ranging from 10 to 30 mW/cm² and lasts for 120 to 180 s;
in a final stage, the laser has a power ranging from 5 to 8 mW/cm² and lasts for 10 to 20 s.

Each working cycle corresponds to a complete cycle in which the laser generator acts on the vascular wall. In the initial stage, the laser having a low power and lasting for a short duration is used to allow the blood vessel to adapt to the laser irradiation, preparing for the subsequent treatment. In the intermediate stage, the power of the laser is increased to heal the torn blood vessel, preventing restenosis after the dilation. In the final stage, the laser having a low power and lasting for a short duration is used for postoperative buffering, reducing the burden on the blood vessel.

If the power of the laser emitted by the laser generator is too low, the repairing result is unsatisfactory, while if the power of the laser is too high, vascular burns may be caused due to excessive heat. Therefore, in some embodiments, the laser has a power ranging from 10 to 15 mW/cm² and lasts for 120 to 180 s during the intermediate stage.

The balloon body 110 can be a smooth bare balloon or a balloon with a different shape. The material of the balloon body 110 needs to have sufficient flexibility and good through-ability such that the balloon body 110 can smoothly reach an intended site. The balloon body 110 also needs to have an excellent processing performance, a good resilience, a fatigue resistance, and a dimensional stability. In some embodiments, the material of the balloon body 110 is one selected from PEBAX, nylon, and TPU. In some further embodiments, the material of the balloon body is PEBAX or nylon.

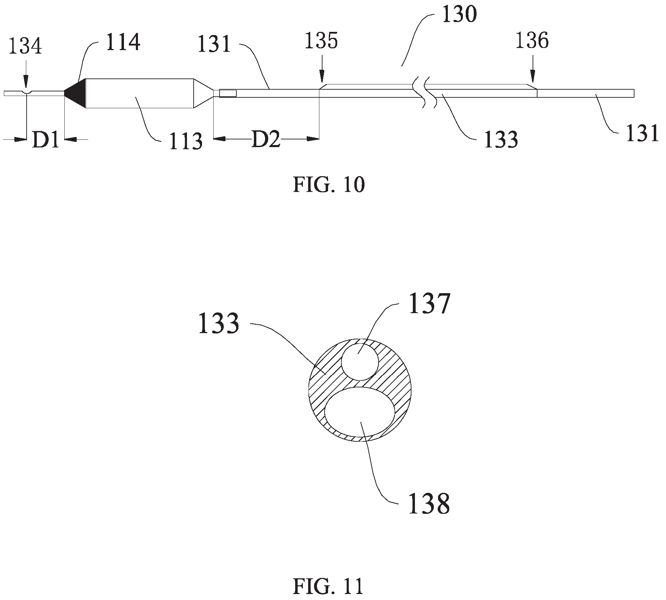

As shown in FIG. 10, the balloon body 110 includes an isodiametric section 113 and tapered sections 114 respectively located at both ends of the isodiametric section 113. A surface of the tapered section 114 located at a distal end of the balloon body 110 (see the black filled area in FIG. 10) is covered with a black coating. The black coating is made of PTFE, mixed-color shellac, or mixed-color ultraviolet adhesive. The tapered sections 114 at both ends of the balloon body 110 are not strictly tapered, but are portions of the balloon body 110 that have shapes approximate to cone due to a change of a diameter of the balloon body 110.

The black coating covered on the distal end of balloon body 110 can serve as a barrier to block laser radiation, preventing the laser from causing burns to unintended sites.

As shown in FIG. 1, the balloon catheter system 100 also includes a stress release tube 150 and a catheter adapter 140. The stress release tube 150 is connected between the tube body 130 and the catheter adapter 140. The catheter adapter 140 is connected to the proximal end of the tube body 130 and has interfaces respectively connected to the fluid channel and the optical fiber channel. After the optical fiber 120 extends out of the optical fiber channel, the optical fiber 120 is connected to the laser generator through an optical fiber joint 160.

The laser emitted from the light emitting section is focused in the area corresponding to the high refractive index ultraviolet adhesive to ensure that the laser applied to the site to be repaired has a sufficient intensity. In some embodiments, a refractive index of the high refractive index ultraviolet adhesive ranges from 1.50 to 1.70. In some further embodiments, the refractive index of the high refractive index ultraviolet adhesive ranges from 1.54 to 1.62.

The tearing of the vascular wall is concentrated in the intended site through the high refractive index ultraviolet adhesive. To avoid complicating tearing of the vascular wall, the high refractive index ultraviolet adhesive is uniformly distributed on the outer periphery of the balloon body 110. The high refractive index ultraviolet adhesive protrudes from the outer surface of the balloon body 110, with a height ranging from 0.20 to 0.25 mm.

As shown in FIG. 2, the high refractive index ultraviolet adhesive is arranged in the form of dot-shaped protrusions 111 arranged in a matrix. A distance between two adjacent dot-shaped protrusions 111 ranges from 3 to 10 mm, and the distance between each dot-shaped protrusion 111 and the adjacent dot-shaped protrusion 111 is the same as each other. Each dot-shaped protrusion 111 is shaped as a hemisphere, and a plane of the hemisphere is connected to the outer surface of the balloon body 110. A height of each dot-shaped protrusion 111 (as shown by H1 in FIG. 2) ranges from 0.20 to 0.25 mm.

As shown in FIG. 3, the high refractive index ultraviolet adhesive is arranged in the form of linear protrusions 112 extending along an axial direction of the balloon body 110 and equidistantly distributed around a circumference of the balloon body 110. The number of the linear protrusions 112 can be 3 to 5. A height of each linear protrusion 112 (as shown by H2 in FIG. 3) ranges from 0.20 to 0.25 mm. A length of each linear protrusion 112 is adapted to that of the isodiametric section 113, and a smooth transition is formed between an end of each linear protrusion 112 and the corresponding tapered section 114.

When the balloon body 110 is filled with fluid through the fluid channel of the tube body 130, the high refractive index ultraviolet adhesive can focus the laser, and the focused laser is applied more intensively to the site to be repaired, avoiding omission of the treatment area.

The high refractive index ultraviolet adhesive not only can concentrate the tearing area of the blood vessel and focus the laser for effective repairing, but also can increase a friction between the balloon body 110 and the vascular wall during the balloon body dilation, thereby reducing the slidable movement of the balloon body 110 relative to the vascular wall, improving the treatment effect, and reducing unnecessary injury to non-treatment areas.

The high refractive index ultraviolet adhesive in a regular distribution can also cut the lesion site, reducing the irregular tearing and dissection of the blood vessel.

As shown in FIG. 1, the tube body 130 includes an inner tube 132 and an outer tube 131 mounted around the inner tube 132. The inner tube 132 and the outer tube 131 of the tube body 130 can be made of the same material or different materials. The inner tube 132 is colorless and transparent, allowing the light transmitted by the optical fiber to radiate outwards through the inner tube 132. The outer tube 131 is black, preventing the light emitted by the optical fiber from reaching unintended sites.

The inner tube 132 of the tube body 130 has a hardness ranging from 35 to 75 D, and the hardness increases continuously or at intervals from the distal end of the tube body 130 to the proximal end of the tube body 130 by a predetermined value.

The inner tube 132 of the tube body 130 includes at least two first unit sections connected in sequence along an axial direction of the tube body 130. For two adjacent first unit sections, a hardness of the first unit section closer to the distal end of the tube body 130 is less than that of the first unit section closer to the proximal end of the tube body 130. The two adjacent first unit sections can be connected by welding.

The number of the first unit sections is 2 to 4.

Each first unit section has the same diameter, or for adjacent two first unit sections, the diameter of the first unit section closer to the distal end of the tube body 130 is less than that of the first unit section closer to the proximal end of the tube body 130. The diameters of the first unit sections gradually increase from the distal end of the tube body 130 to the proximal end of the tube body 130 along the axial direction of the tube body 130, which can ensure the maneuverability of the tube body 130 at the proximal end thereof, and also allows the inner tube 132 to have a smaller diameter at the distal end thereof, such that the inner tube 132 can extend into narrower blood vessels, especially cerebral blood vessels.

The first unit sections have the same axial length or different axial lengths.

The axial length of the first unit section closest to the proximal end of the tube body 130 ranges from 20 to 30 cm.

Each first unit section adopts a single-layer tube or a multi-layer tube. The single-layer tube can be made of one selected from PEBAX, nylon, and TPU. The layers of the multi-layer tube can slide relative to each other, and each layer is independently made of one selected from PEBAX, nylon, and PE.

In some embodiments, each first unit section adopts a single-layer tube made of nylon or PEBAX.

In some embodiments, each first unit section adopts a multi-layer tube; the layers of the multi-layer tube can be made of the same or different materials, and the layers of the multi-layer tube have the same thickness or different thicknesses.

In some embodiments, each first unit section adopts a three-layer tube, and the layers of the three-layer tube can slide relatively to each other. From outside to inside, thicknesses of the three layers are 0.04 mm, 0.05 mm, and 0.01 mm respectively, and the three layers are respectively made of HDPE, LDPE, and nylon.

The inner tube 132 of the tube body 130 includes a proximal inner tube and a distal inner tube connected to the proximal inner tube along the axial direction of the tube body 130. A hardness of the proximal inner tube ranges from 55 to 75 D, and a hardness of the distal inner tube ranges from 35 to 55 D.

In some embodiments, the proximal inner tube adopts a three-layer tube, and the distal inner tube adopts a three-layer tube or a single-layer tube.

In some embodiments, both the proximal inner tube and the distal inner tube adopt a three-layer tube, and each layer of the three-layer tube is made of PEBAX.

The outer tube 131 of the tube body 130 includes at least two second unit sections connected in sequence along the axial direction of the tube body 130. For two adjacent second unit sections, a hardness of the second unit section closer to the distal end of the tube body 130 is less than that of the second unit section closer to the proximal end of the tube body 130. The two adjacent second unit sections are connected by welding.

The number of the second unit sections is 2 to 4.

The second unit sections can have the same diameter, or for two adjacent second unit sections, a diameter of the second unit section closer to the distal end of the tube body 130 is less than that of the second unit section closer to the proximal end of the tube body 130. The diameters of the second unit sections gradually increase from the distal end to the proximal end of the tube body 130 along the axial direction of the tube body 130, which can ensure the maneuverability of the tube body 130 at the proximal end thereof, and also allows the inner tube 132 to have a smaller diameter at the distal end of the tube body 130, such that the inner tube 132 can extend into narrower blood vessels, especially cerebral blood vessels.

The second unit sections have the same axial length or different axial lengths.

In some embodiments, each second unit section adopts a single-layer tube or a multi-layer tube. The single-layer tube can be made of PEBAX or nylon. The layers of the multi-layer tube can slide relative to each other, and each layer is independently made of PEBAX or nylon.

In some embodiments, each second unit section adopts a multi-layer tube, the layers of the multi-layer tube can be made of the same material or different materials, and the layers of the multi-layer tube have the same thickness or different thicknesses.

In the present disclosure, by selecting appropriate material for the balloon body 110, the inner tube 132 and the outer tube 131, the balloon body 110, the inner tube 132, and the outer tube 131 can form a delivery system that is relatively more flexible, which can balance the increase in a hardness of the delivery system due to the addition of the optical fiber, such that the delivery system can have an ideal combination of radial support and flexibility.

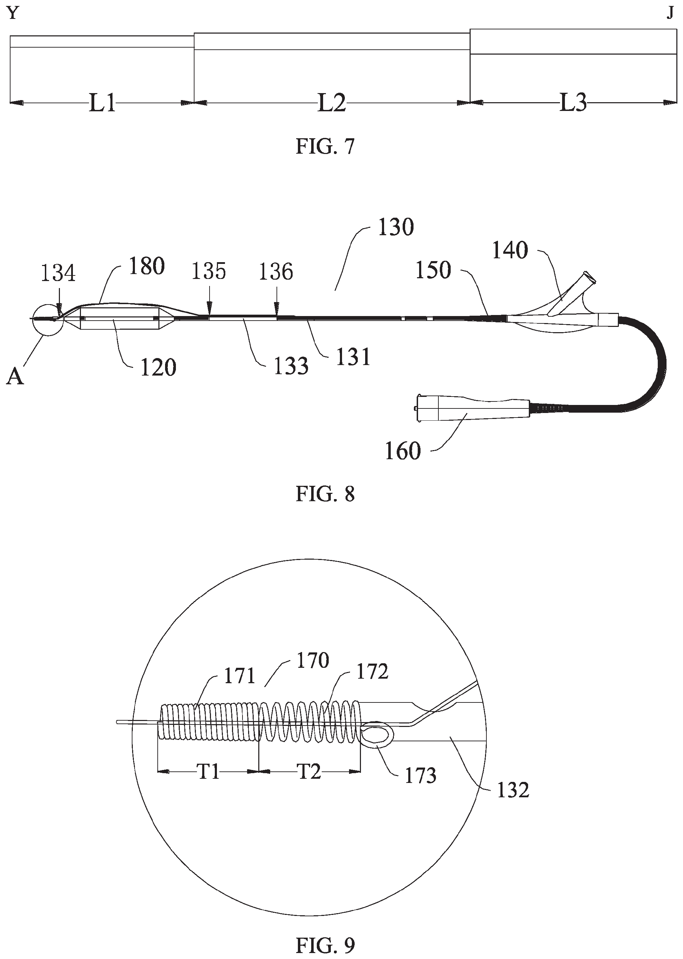

As shown in FIG. 7, Y represents the distal end of the tube body 130, and J represents the proximal end of the tube body 130. According to the order from the distal end to the proximal end, the outer tube 131 includes a second unit section L1, a second unit section L2, and a second unit section L3 connected in sequence along the axial direction of the tube body 130. The second unit sections are connected by welding and are arranged coaxially. A diameter of the second unit section L1 is 0.85 mm, and an axial length of the second unit section L1 is 120 mm. A diameter of the second unit section L2 is 1.10 mm, and an axial length of the second unit section L2 is 140 mm. A diameter of the second unit section L3 is 1.10 mm, and an axial length of the second unit section L3 is 1300 mm. Both the second unit section L1 and the second unit section L2 are made of PEBAX, and the second unit section L3 is made of nylon.

The structure of the outer tube 131 is shown in FIG. 7. The structure of the inner tube 132 is the same as that of the outer tube 131, but the materials and diameters of the inner tube 132 are different from those of the outer tube 131. When the outer tube 131 of the tube body 130 includes multiple second unit sections as shown in FIG. 7, correspondingly, the inner tube 132 also includes multiple first unit sections. The number of the first unit sections is the same as that of the second unit sections, and the axial length of each first unit section is the same as that of each second unit section. From the distal end to the proximal end, the three first unit sections of the inner tube have the same diameter of 0.54 mm, different hardness of 55 D, 63 D, and 70 D in turn, and are respectively made of PEBAX, PEBAX, and PA in turn.

When the outer tube 131 is a single-diameter tube, the inner tube 132 is also a single-diameter tube, and the structure of the inner tube 132 is the same as that of the outer tube 131.

As shown in FIGS. 8 and 9, an end portion of a distal end of the inner tube 132 is welded with an elastic member 170. The elastic member 170 includes a bare section 171 (corresponding to the section T1 in FIG. 9) and a welding section 172 (corresponding to the section T2 in FIG. 9) along the axial direction of the tube body 130. A ratio of an axial length of the bare section 171 to that of the welding section 172 ranges from 1:0.8 to 1:1.2. A diameter of the elastic member 170 gradually increases from a distal end of the bare section 171 to a proximal end of the welding section 172.

At the distal end of the tube body 130, the inner tube 132 intersects with the distal end of the balloon body 110, and the distal end of the inner tube 132 is welded with the elastic member 170. The elastic member 170 may also be welded to the distal end of the balloon body 110. The elastic member 170 is generally located at the distal end of the inner tube 132. The welding section 172 overlaps with the distal end of the inner tube 132 and is connected to the distal end of the inner tube 132 by welding. The bare section 171 is directly exposed to the external environment, neither located in a wall of the inner tube 132 nor overlapping with other components.

The elastic member 170 has a certain elasticity. As the part of the tube body 130 that firstly extends into the blood vessel, the elastic member 170 is deformable when encountering an obstruction inside the blood vessel, which can avoid puncturing the blood vessel. Meanwhile, based on the resilience of the elastic member 170, the elastic member 170 can restore to its original shape after the external force is removed.

The elastic member 170 is a spiral spring, which is formed by spirally winding of a metal wire having a diameter ranging from 0.04 to 0.1 mm. In some embodiments, the spiral spring is formed by spirally winding of a metal wire with a diameter ranging from 0.05 to 0.06 mm. The metal wire is made of one selected from platinum-tungsten, platinum-iridium, stainless steel, gold, and nickel-titanium.

The elastic member 170 is made of metal wires, which is opaque, thus, the elastic member 170 can shield the light emitted by the optical fiber at the distal end of the balloon body 110 to avoid injury to unintended sites due to the laser radiation.

A thread pitch of the bare section 171 is equal to a diameter of the metal wire, and a thread pitch of the welding section 172 is 1 to 3 times the diameter of the metal wire. In some embodiments, the thread pitch of the welding section 172 is 1.5 to 2 times the diameter of the metal wire.

The bare section 171 is exposed to the external environment. Since the bare section 171 has a relatively-smaller thread pitch, the bare section 171 can improve the resilience of the elastic member 170. However, since the welding section 172 needs to be connected to the wall of the inner tube 132, the thread pitch of the welding section 172 needs to be set to be relatively larger, such that the welding section 172 can avoid the situation that the flexibility of the wall of the inner tube 132 is excessively reduced, which may easily cause injury to the vascular wall.

The metal wire has a developing function, and the elastic member 170 can also serve as a developing component to indicate the position of the balloon body 110 in the human body.

An axial length of the elastic member 170 ranges from 2 to 5 mm. In some embodiments, the axial length of the elastic member 170 ranges from 3 to 5 mm.

The axial length of the elastic member 170 needs to be appropriate. A too-long axial length is adverse to the restoring of the elastic member 170, and a too-short axial length may prevent the elastic member 170 from playing a role in guiding the balloon body 110 forwards along the blood vessel.

The axial length of the bare section 171 of the elastic member 170 ranges from 0.5 to 2 mm, and the axial length of the welding section 172 ranges from 1 to 3 mm. In some embodiments, the axial length of the bare section 171 ranges from 1 to 2 mm, and the axial length of the welding section 172 ranges from 2 to 3 mm.

A diameter of the bare section 171 ranges from 0.50 to 0.70 mm, and a diameter of the welding section 172 ranges from 0.60 to 1.0mm. In some embodiments, the diameter of the bare section 171 ranges from 0.5 to 0.6 mm, and the diameter of the welding section 172 ranges from 0.6 to 0.7 mm.

The diameter of the bare section 171 needs to allow the the bare section 171 to enter a thin blood vessel without losing the resilience of the bare section 171, and the diameter of the welding section 172 matches with that of the inner tube 132.

A ratio of a diameter of the distal end of the bare section 171 to that of the proximal end of the welding section 172 ranges from 1:1 to 1:1.5. In some embodiments, the ratio ranges from 1:1 to 1:1.2.

The proximal end of the welding section 172 forms a welding ring 173 welded to the wall of the inner tube 132 by bending the metal wire forming the spiral spring. The welding ring 173 can increase a welding area and ensure the firmness of the welding connection.

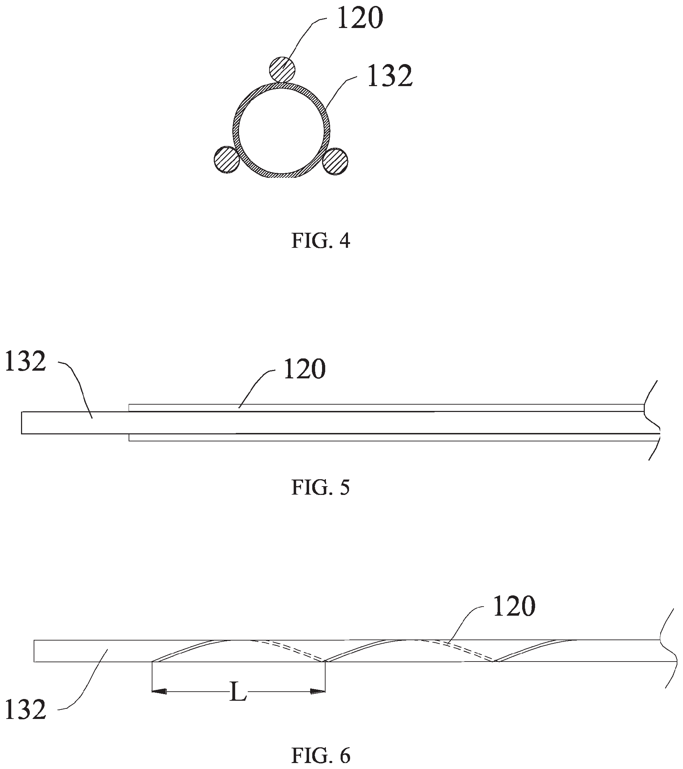

As shown in FIGS. 4, 5, and 6, the balloon catheter system includes multiple optical fibers 120 uniformly distributed around an axis of the tube body 130.

As shown in FIG. 1, the tube body 130 includes the inner tube 132 and the outer tube 131 mounted around the inner tube 132. A radial gap between the inner tube 132 and the outer tube 131 forms the optical fiber channel. As shown in FIG. 5, each optical fiber 120 is arranged in parallel with the inner tube 132, or as shown in FIG. 6, the optical fibers 120 are arranged by wrapping around the inner tube 132. In the embodiment that the optical fibers 120 are arranged by wrapping around the inner tube 132, each wrapping unit of the optical fiber 120 may have an axial length L of 10 mm. In some embodiments, each optical fiber 120 is parallel to the inner tube 132. The optical fibers 120 can be fixed or not fixed to the inner tube 132.

The optical fiber 120 can be made of plastic or quartz. In some embodiments, the optical fiber 120 is made of plastic. A diameter of the optical fiber 120 ranges from 0.125 to 0.25 mm; in some embodiments, the diameter of the optical fiber 120 is 0.125 mm.

The number of the optical fibers 120 can be 1 to 13, which can be 3 to 6 in some embodiments. In an embodiment, the number of the optical fibers 120 is 3. As shown in FIG. 4, the three optical fibers 120 are arranged equidistantly around the inner tube 132, and two adjacent optical fibers 120 correspond to an angle of 120° of the inner tube 132.

The light emitting section of the optical fiber 120 is produced by removing a cladding from a portion of the optical fiber 120 located within the balloon body 110, and a length of the removed cladding is equal to the axial length of the balloon body 110. The cladding can be removed physically (e.g., sandblasting, grinding, and scraping) or chemically.

As shown in FIGS. 8 and 10, the tube body 130 includes the inner tube 132 and the outer tube 131 mounted around the inner tube 132. The distal end of the balloon body 110 is in sealed connection with an outer wall of the inner tube 132, and a proximal end of the balloon body 110 is in sealed connection with the outer tube 131. The radial gap between the inner tube 132 and the outer tube 131 form the fluid channel. A lumen of the inner tube 132 forms the optical fiber channel.

At least one section of the tube body 130 along the axial direction thereof is a double-lumen tube 133, which includes a fluid channel 138 and a guide wire channel 137. The fluid channel 138 and the guide wire channel 137 are parallel to each other and are isolated from each other. The fluid channel 138 communicates with the radial gap between the inner tube 132 and the outer tube 131. As shown in FIG. 11, a cross-sectional area of the fluid channel 138 is greater than that of the guide wire channel 137.

The inner tube 132 extends out of the distal end of the balloon body 110, and a first guide wire port 134 is formed in a tube wall of a portion of the inner tube 132 extending out of the distal end of the balloon body 110. Both ends of the guide wire channel 137 respectively form a second guide wire port 135 and a third guide wire port 136. A direction of the guide wire 180 is shown in FIG. 8. In the use state, the guide wire 180 enters the guide wire channel 137 of the double-lumen tube through the third guide wire port 136, extends out of the balloon body 110 through the second guide wire port 135, and then enters the lumen of the inner tube 132 through the first guide wire port 134. A diameter of the lumen of the inner tube 132 ranges from 0.54 to 0.80 mm. In some embodiments, the diameter of the lumen of the inner tube 132 ranges from 0.54 to 0.74 mm.

As shown in FIG. 10, the first guide wire port 134, the second guide wire port 135, and the third guide wire port 136 are aligned in the axial direction of the tube body 130. A distance D1 between the first guide wire port 134 and the distal end of the balloon body 110 ranges from 5 to 20 mm, and a distance D2 between the second guide wire port 135 and the proximal end of the balloon body 110 ranges from10 to 30 mm. In some embodiments, the distance D1 between the first guide wire port 134 and the distal end of the balloon body 110 ranges from 5 to 15 mm, and the distance D2 between the second guide wire port 135 and the proximal end of the balloon body 110 ranges from 10-30 mm.

The guide wire is guided to extend out of balloon body 110 through the double-lumen tube 133, while the optical fiber 120 is placed inside the lumen of the inner tube 132 which originally forms a guide wire channel. The optical fiber 120 and the inner tube 132 are coaxially arranged (within an acceptable margin of error, and it is not necessary to require that the optical fiber 120 and the inner tube 132 are strictly coaxial), which allows the light emitted by the optical fiber to illuminate the vascular wall more uniformly.

The path of the guide wire 180 is partially located outside balloon body 110, thus, the lesion site can be cut to reduce irregular tearing and dissections of the blood vessel.

To improve the treatment effect, the outer surface of the balloon body 110 is coated with at least one selected from an anti-proliferative drug, a drug that induce collagen or elastin cross-linking, and an anti-vasospasm drug.

The drug coating on the outer surface of the balloon body 110 can act on the injury site, which can achieve corresponding efficacy and facilitate comprehensive repairing of the injury site. In some embodiments, the drug is paclitaxel or rapamycin.

In some embodiments, the outer surface of balloon body 110 is provided with an implantable vascular stent.

When being used to provide laser repairing, the balloon body 110 can be used to implant the vascular stent into the human body at the same time, achieving two treatment objectives at once. The vascular stent generally has a compressible mesh structures that does not significantly block the light during the laser repairing process, thus, the effect of the laser repairing may not be affected by the vascular stent. Moreover, based on the result of the laser repairing, after the vascular stent is implanted, problems like neointimal hyperplasia may not be easily caused.

The technical features of the above embodiments can be combined in any way. To simplify the description, not all possible combinations of the technical features in the above embodiments have been described. However, as long as the combination of these technical features does not contradict each other, it should be considered within the scope of this specification.

The above embodiments only express several implementation methods of this application, and the description is relatively specific and detailed. However, it should not be understood as a limitation on the scope of the invention patent. It should be pointed out that, for those skilled in the art, they can also make several modifications and improvements without departing from the concept of this application, and all these are within the scope of protection of this application. Therefore, the scope of protection of this application should be based on the attached claims.

## Claims

1. A balloon catheter system with repair function, comprising:
a tube body, having a distal end and a proximal end opposite to the distal end, and the tube body at least forming a fluid channel and an optical fiber channel;
a balloon body, located at an outer periphery of the distal end of the tube body and communicating with the fluid channel;
an optical fiber, arranged in the optical fiber channel and having a light emitting section extending to a position of the balloon body; and
a laser generator, connected to a proximal end of the optical fiber and emitting a laser having a repair function with a wavelength ranging from 400 to 750 nm.

2. The balloon catheter system according to claim 1, wherein a power of the laser emitted by the laser generator ranges from 10 to 30 mW.

3. The balloon catheter system according to claim 1, wherein the laser having a predetermined wavelength is emitted by the laser generator in the following sequence in each working cycle:
in an initial stage, the laser has a power ranging from 5 to 8 mW/cm² and lasts for 10 to 20 s;
in an intermediate stage, the laser has a power ranging from 10 to 30 mW/cm² and lasts for 120 to 180 s; and
in a final stage, the laser has a power ranging from 5 to 8 mW/cm² and lasts for 10 to 20 s.

4. The balloon catheter system according to claim 1, wherein the balloon body comprises an isodiametric section and two tapered sections respectively located at both ends of the isodiametric section, and a surface of each tapered section at a distal end of the balloon body is covered with a black coating.

5. The balloon catheter system according to claim 1, wherein a refractive index of the high refractive index ultraviolet adhesive ranges from 1.50 to 1.70.

6. The balloon catheter system according to claim 1, wherein high refractive index ultraviolet adhesive is attached to an outer periphery of the balloon body distributed at intervals, and the high refractive index ultraviolet adhesive protrudes from a surface of the balloon body at a height ranging from 0.20 to 0.25 mm.

7. The balloon catheter system according to claim 6, wherein the high refractive index ultraviolet adhesive is regularly distributed on the outer periphery of the balloon body.

8. The balloon catheter system according to claim 6, wherein the high refractive index ultraviolet adhesive is arranged in the form of dot-shaped protrusions in a matrix, and a distance between two adjacent spots ranges from 3 to 10 mm.

9. The balloon catheter system according to claim 4, wherein the high refractive index ultraviolet adhesive is arranged in the form of linear protrusions extending along an axial direction of the balloon body and equidistantly distributed around a circumference of the balloon body, and a number of the linear protrusions ranges from 3 to 10.

10. The balloon catheter system according to claim 9, wherein a length of the linear protrusions matches a length of the isodiametric section.

11. The balloon catheter system according to claim 1, wherein the tube body comprises an inner tube and an outer tube mounted around the inner tube, the outer tube of the tube body is black, and the inner tube of the tube body is colorless and transparent; the inner tube of the tube body has a hardness ranging from 35 to 75D, and the hardness of the inner tube increases continuously or at intervals from the distal end to the proximal end of the tube body by a predetermined value.

12. The balloon catheter system according to claim 11, wherein the inner tube of the tube body comprises at least two first unit sections connected in sequence along an axial direction of the tube body, and for two adjacent first unit sections, a hardness of the first unit section closer to the distal end of the tube body is less than a hardness of the first unit section closer to the proximal end of the tube body.

13. The balloon catheter system according to claim 12, wherein a number of the first unit sections is 2 to 4.

14. The balloon catheter system according to claim 12, wherein a diameter of each first unit section is the same as each other; or
wherein for two adjacent first unit sections, a diameter of the first unit section closer to the distal end of the tube body is less than a diameter of the first unit section closer to the proximal end of the tube body.

15. The balloon catheter system according to claim 12, wherein an axial length of the first unit section closest to the proximal end of the tube body ranges from 20 to 30 cm.

16. The balloon catheter system according to claim 12, wherein each first unit section is a single-layer tube or a multi-layer tube, the single-layer tube is made of one selected from PEBAX, nylon and TPU, and each layer of the multi-layer tube is independently made of one selected from PEBAX, nylon and PE.

17. The balloon catheter system according to claim 11, wherein the inner tube of the tube body comprises a proximal inner tube and a distal inner tube connected to the proximal inner tube along the axial direction of the tube body; a hardness of the proximal inner tube ranges from 55 to 75D, and a hardness of the distal inner tube ranges from 35 to 55 D.

18. The balloon catheter system according to claim 11, wherein the outer tube of the tube body comprises at least two second unit sections connected in sequence along the axial direction of the tube body; for the two adjacent second unit sections, a hardness of the second unit section closer to the distal end of the tube body is less than that of the second unit section closer to the proximal end of the tube body.

19. The balloon catheter system according to claim 18, wherein and a number of the second unit sections is 2 to 4.

20. The balloon catheter system according to claim 18, wherein a diameter of each second unit section is the same as each other; or, for the two adjacent second unit sections, a diameter of the second unit section closer to the distal end of the tube body is less than that of the second unit section closer to the proximal end of the tube body.

21. The balloon catheter system according to claim 18, wherein each second unit section adopts a single-layer tube or a multi-layer tube, the single-layer tube is made of one selected from PEBAX or nylon, and each layer of the multi-layer tube is independently made of PEBAX or nylon.

22. The balloon catheter system according to claim 11, wherein an end portion of a distal end of the inner tube is welded with an elastic member, and the elastic member comprises a bare section and a welding section arranged along the axial direction of the tube body; a ratio of an axial length of the bare section to that of the welding section ranges from 1:0.8 to 1:1.2, and a diameter of the elastic member gradually increases from a distal end of the bare section to a proximal end of the welding section.

23. The balloon catheter system according to claim 22, wherein the elastic member is a spiral spring formed by spirally winding a metal wire with a diameter ranging from 0.04 to 0.1 mm.

24. The balloon catheter system according to claim 23, wherein the metal wire is made of one selected from platinum-tungsten, platinum-iridium, stainless steel, gold, and nickel-titanium.

25. The balloon catheter system according to claim 23, wherein a thread pitch of the bare section is equal to a diameter of the metal wire, and a thread pitch of the welding section is 1 to 3 times the diameter of the metal wire.

26. The balloon catheter system according to claim 22, wherein an axial length of the elastic member ranges from 2 to 5 mm.

27. The balloon catheter system according to claim 22,
wherein an axial length of the bare section of the elastic member ranges from 0.5 to 2 mm, an axial length of the welding section ranges from 1 to 3 mm.

28. The balloon catheter system according to claim 22,
wherein a diameter of the bare section ranges from 0.50 to 0.70 mm, and a diameter of the welding section ranges from 0.60 to 1.0 mm.

29. The balloon catheter system according to claim 22, wherein a ratio of a distal diameter of the bare section to a proximal diameter of the welding section ranges from 1:1 to 1:1.5.

30. The balloon catheter system according to claim 22, wherein the proximal end of the welding section forms a welding ring welded to the wall of the inner tube by bending the metal wire forming the spiral spring.

31. The balloon catheter system according to claim 1, wherein the tube body comprises an inner tube and an outer tube mounted around the inner tube, a distal end of the balloon body is in sealed connection with an outer wall of the inner tube, and a proximal end of the balloon body in sealed connection with the outer tube, a radial gap between the inner tube and the outer tube forms the optical fiber channel, and each optical fiber is arranged in parallel with the inner tube or by wrapping around the inner tube.

32. The balloon catheter system according to claim 31, wherein the balloon catheter system comprises a plurality of the optical fibers, and the optical fibers are uniformly distributed around an axis of the tube body.

33. The balloon catheter system according to claim 31, wherein a diameter of each optical fiber ranges from 0.125 to 0.25 mm.

34. The balloon catheter system according to claim 1, wherein the tube body comprises an inner tube and an outer tube mounted around the inner tube, a distal end of the balloon body is in sealed connection with the outer wall of the inner tube, and a proximal end of the balloon body is in sealed connection with the outer tube; a radial gap between the inner tube and the outer tube forms a fluid channel, and the inner tube lumen forms the optical fiber channel.

35. The balloon catheter system according to claim 34, wherein at least one section of the tube body along an axial direction thereof is a double-lumen tube, and the double-lumen tube comprises the fluid channel and a guide wire channel isolated from the fluid channel, and the fluid channel communicates with the radial gap between the inner tube and the outer tube.

36. The balloon catheter system according to claim 35, wherein a first guide wire port is formed in a tube wall of a portion of the inner tube extending out of a distal end of the balloon body; two ends of the guide wire channel respectively form a second guide wire port and a third guide wire port; the guide wire passes through the first guide wire port, the second guide wire port, and the third guide wire port, and a portion of the guide wire between the first guide wire port and the second guide wire port is located outside the balloon body.

37. The balloon catheter system according to claim 1, wherein an outer surface of the balloon body is coated with at least one selected from an anti-proliferative drug, a drug that induce collagen or elastin cross-linking, and an anti-vasospasm drug.

38. The balloon catheter system according to claim 37, wherein the drug is paclitaxel or rapamycin.

39. The balloon catheter system according to claim 1, wherein the balloon body is provided with an implantable vascular stent on the outer surface thereof.
